(19) Europäisches Patentamt
European Patent Office
Office européen des brevets

(11) EP 0 846 460 A1

(12) **DEMANDE DE BREVET EUROPEEN**

(43) Date de publication:
**10.06.1998 Bulletin 1998/24**

(51) Int Cl.6: **A61K 7/00**, A61K 7/06

(21) Numéro de dépôt: **97402847.4**

(22) Date de dépôt: **26.11.1997**

(84) Etats contractants désignés:
**AT BE CH DE DK ES FI FR GB GR IE IT LI LU MC NL PT SE**

(30) Priorité: **04.12.1996 FR 9614329**

(71) Demandeur: **L'OREAL**
**75008 Paris (FR)**

(72) Inventeurs:
- **Samain, Henri M.**
**91570 Bievres (FR)**
- **Minou, Patrick M.**
**94200 Courbevoie (FR)**

(74) Mandataire: **Andral, Christophe André Louis**
**L'OREAL**
**Centre de Recherche Charles Zviak**
**Département Propriété Industrielle**
**90, rue du Général Roguet**
**92583 Clichy Cedex (FR)**

(54) **Dispositif aérosol à base de compositions alcooliques de matériaux fixants**

(57) La présente invention concerne un dispositif aérosol constitué par un réservoir contenant une composition aérosol constituée par d'une part une phase liquide (ou jus) contenant au moins un matériau fixant dans un solvant approprié et d'autre part un propulseur, et un moyen de distribution de ladite composition aérosol, le matériau fixant ayant une température de transition vitreuse (Tg) supérieure ou égale à 30 °C, et le dispositif étant approprié pour obtenir un débit en matière sèche compris entre environ 4 et 17 mg/s et un pouvoir mouillant supérieur ou égal à 50 mg/s.

Elle concerne également un procédé de traitement des fibres kératiniques, dans lequel on applique sur lesdites fibres une composition comprenant un matériau fixant ayant une température de transition vitreuse (Tg) supérieure ou égale à 30 °C, au moyen d'un dispositif approprié pour obtenir un débit en matière sèche compris entre environ 4 et 17 mg/s et un pouvoir mouillant supérieur ou égal à 50 mg/s.

EP 0 846 460 A1

## Description

La présente invention concerne de nouveaux dispositifs aérosols destinés à fixer les chevelures.

Les produits capillaires pour la mise en forme et/ou le maintien de la coiffure les plus répandus sur le marché de la cosmétique sont des compositions à pulvériser essentiellement constituées d'une solution le plus souvent alcoolique ou aqueuse et d'un ou plusieurs matériaux, généralement des résines polymères, dont la fonction est de former des soudures entre les cheveux, appelés encore matériaux fixants, en mélange avec divers adjuvants cosmétiques. Cette solution est généralement conditionnée soit dans un récipient aérosol approprié mis sous pression à l'aide d'un propulseur, soit dans un flacon pompe.

Les matériaux fixants sont généralement des polymères fixants, c'est à dire des polymères filmogènes solubles dans l'eau et dans l'alcool, tels que la polyvinylpyrrolidone, les copolymères de vinylpyrrolidone/acétate de vinyle, décrits notamment dans les brevets US 3 929 735 et US 3 770 683, les copolymères d'acétate de vinyle/acide crotonique, les résines acryliques anioniques ou amphotères. Ces matériaux permettent d'obtenir facilement l'effet fixant, en revanche, après brossage ou peignage, les cheveux présentent dans les conditions usuelles de laquage un aspect raidi et un toucher rêche, voire collant.

Ces inconvénients sont liés à plusieurs paramètres, parmi lesquels on peut citer la nature du ou des polymères fixants, ou encore à la nature des soudures. Pour remédier à ces inconvénients on peut donc agir sur ces deux paramètres, sans toutefois diminuer l'effet fixant recherché. Pour améliorer les propriétés cosmétiques des matériaux fixants, il a surtout été proposé d'associer différents polymères (WO 94/12148, WO 96/06592, US 5 158 762).

La Demanderesse a maintenant trouvé qu'en sélectionnant de manière approprié les polymères fixants et les paramètres de diffusion des compositions, il était possible de diminuer la taille des soudures.

La présente invention concerne donc un nouveau dispositif aérosol destiné à fixer les chevelures, permettant de diminuer la taille des soudures, et donc apporter d'excellentes propriétés cosmétiques telles que la douceur, le démêlage et le toucher, tout en conservant de bonnes qualités de fixation et/ou de mise en forme la coiffure.

Le dispositif aérosol selon l'invention est constitué par un récipient contenant une composition aérosol constituée d'une part par une phase liquide (ou jus) contenant au moins un matériau fixant dans un solvant approprié et d'autre part un propulseur, et un moyen de distribution de ladite composition aérosol, le matériau fixant ayant une température de transition vitreuse (Tg) supérieure ou égale à 30 °C, et le dispositif étant approprié pour obtenir un débit en matière sèche compris entre environ 4 et 17 mg/s et un pouvoir mouillant supérieur ou égal à 50 mg/s.

Le moyen de distribution est généralement constitué par une valve de distribution commandée par une tête de distribution, elle même comprenant une buse par laquelle la composition aérosol est vaporisée.

Les notions de «Tg», « débit en matière sèche » et « pouvoir mouillant » telles qu'on les entend selon la présente invention sont définies ci-dessous.

Par température de transition vitreuse (Tg), on entend selon la présente invention la Tg du matériau fixant dans l'extrait sec, l'extrait sec étant constitué par l'ensemble des matériaux non volatiles dans le jus, ou matière sèche.

Selon la présente invention, le débit en matière ($D_{MS}$) sèche correspond ne la quantité d'extrait sec qui sort du dispositif aérosol par unité de temps. Ce débit en matière sèche est exprimé en mg/s et est calculé en multipliant la concentration en matière sèche dans la composition aérosol ($C_{MS}$) par le débit de la composition aérosol à la sortie de la buse ($D_{CA}$):

$$D_{MS} = C_{MS} \times D_{CA}.$$

La concentration en matière sèche dans la composition aérosol ($C_{MS}$) correspond à la quantité de matière sèche ramenée à 100g de composition aérosol (jus + propulseur). La concentration en matière sèche est exprimée en pourcentage et est mesurée après pulvérisation par évaporation des composants volatils du résidu de pulvérisation pendant 1 heure 30 à 105 °C.

Le débit en composition aérosol ($D_{CA}$) correspond à la quantité de composition aérosol (jus + propulseur) sortant du dispositif aérosol par unité de temps. Il est exprimé en mg/s et est mesuré par la différence entre le poids de l'aérosol avant ($M_0$) et après ($M_1$) 10 secondes de vaporisation:

$$D_{CA} = (M_0 - M_1) / 10.$$

Selon la présente invention, le pouvoir mouillant correspond à la quantité de produit reçue par une feuille de matière plastique disposée à une distance de 35 cm de la buse du dispositif aérosol pendant une unité de temps donnée. Le produit est alors constitué par la matière sèche, plus une partie du solvant non évaporé en cours du trajet, plus éventuellement une partie du propulseur non évaporé. Ce pouvoir mouillant est exprimé en mg/s, et est mesuré selon

l'invention par la méthode suivante:

- on suspend verticalement une feuille de matière plastique de dimensions 21 cm x 23 cm à une balance de précision (1/1000), la feuille étant reliée à la balance par le bord supérieur (généralement par un crochet de la balance inséré dans une perforation disposée au centre de la largeur et à 1 cm du bord supérieur), et maintenue verticale par l'application d'un poids centré sur le bord inférieur (généralement par une pince fixée et centrée sur le bord inférieur);
- on place une cale derrière le bord inférieur de la feuille pour maintenir la feuille verticale lors de l'impact du produit;
- on dispose verticalement le dispositif aérosol de manière que la buse de diffusion de la composition soit disposée au centre et à 35 cm de la feuille verticale, pour une vaporisation du produit perpendiculaire à la feuille;
- on vaporise la composition pendant 5 secondes;
- on mesure la quantité de produit reçue sur la feuille verticale dès la fin de la vaporisation.

Pour plus de précision, on peut employer un dispositif approprié comprenant un moyen support du dispositif aérosol, et des moyens permettant le réglage tridimensionnel de la position de la buse par rapport à la feuille verticale. Ce dispositif peut être également équipé d'un dispositif pneumatique de contrôle du spray (déclenchement et durée), de manière à contrôler avec précision la durée de la vaporisation. L'ensemble peut être contrôlé par un ordinateur.

Pour éviter les perturbations environnementales, le trajet du produit entre la buse et la feuille sera avantageusement protégé horizontalement et verticalement par les parois d'un tunnel de dimensions appropriées.

Enfin, la vaporisation du produit est avantageusement effectuée sous atmosphère contrôlée, de manière préférentielle à une température de 20°C et une humidité relative de 50%.

Selon un mode préférentiel de réalisation de l'invention, le dispositif aérosol selon l'invention est approprié pour obtenir un débit en matière sèche inférieur à 16 mg/s, de préférence compris entre 6 et 15 mg/s.

De manière avantageuse, le dispositif aérosol selon l'invention est approprié pour obtenir un pouvoir mouillant compris entre 50 mg/s et 125 mg/s.

Les caractéristiques de débit en matière sèche et de pouvoir mouillant des dispositifs aérosols selon l'invention dépendent d'une part de la composition aérosol, et d'autre part du moyen de distribution, les deux devant être appropriés pour obtenir les caractéristiques recherchées. Parmi les paramètres susceptibles d'influencer ces caractéristiques, on citera plus particulièrement la concentration en matière sèche ($C_{MS}$), le débit en composition aérosol ($D_{CA}$) et la phase de la composition aérosol.

D'une manière avantageuse, la concentration en matière sèche ($C_{MS}$) est comprise entre 0,4 et 5% en poids par rapport au poids total de la composition aérosol (jus + propulseur), de préférence comprise entre 0,6 et 3,25% en poids.

Le débit en composition aérosol ($D_{CA}$) sera alors approprié pour obtenir un débit en matière sèche ($D_{MS}$) tel que défini ci-dessus. De manière préférentielle, le $D_{MS}$ sera compris entre 500 et 700 mg/s, plus préférentiellement voisin de 550 mg/s.

La phase de la composition aérosol est de préférence une phase longue, c'est à dire que le rapport pondéral jus/propulseur est supérieur à 1, plus préférentiellement compris entre 1,2 et 3.

Le matériau fixant est essentiellement constitué par au moins un polymère fixant, seul ou en combinaison avec des additifs cosmétiques usuels, par exemple des plastifiants, ou des agents neutralisants.

Selon l'invention, on peut utiliser tout polymère fixant connu en soi. On peut utiliser en particulier un polymère fixant choisi parmi les polymères anioniques, cationiques, amphotères, non ioniques et leurs mélanges. Les polymères fixants peuvent être utilisés sous forme solubilisée ou sous forme de dispersions de particules solides de polymère.

Les polymères fixants cationiques utilisables selon la présente invention sont de préférence choisis parmi les polymères comportant des groupements amine primaire, secondaire, tertiaire et/ou quaternaire faisant partie de la chaîne polymère ou directement reliés à celle-ci, et ayant un poids moléculaire compris entre 500 et environ 5.000.000 et de préférence entre 1000 et 3.000.000.

Selon la présente invention, les polymères fixants sont de préférence des polymères anioniques ou amphotères.

Les polymères fixants anioniques généralement utilisés sont des polymères comportant des groupements dérivés d'acide carboxylique, sulfonique ou phosphorique et ont un poids moléculaire moyen en poids compris entre environ 500 et 5.000.000.

1) Les groupements carboxyliques sont apportés par des monomères mono ou diacides carboxyliques insaturés tels que ceux répondant à la formule :

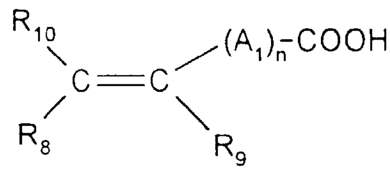

(IV)

dans laquelle n est un nombre entier de 0 à 10, $A_1$ désigne un groupement méthylène, éventuellement relié à l'atome de carbone du groupement insaturé ou au groupement méthylène voisin lorsque n est supérieur à 1 par l'intermédiaire d'un hétéroatome tel que oxygène ou soufre, $R_{10}$ désigne un atome d'hydrogène, un groupement phényle ou benzyle, $R_8$ désigne un atome d'hydrogène, un groupement alkyle inférieur ou carboxyle, Rg désigne un atome d'hydrogène, un groupement alkyle inférieur, un groupement $-CH_2-COOH$, phényle ou benzyle ; Dans la formule précitée un radical alkyle inférieur désigne de préférence un groupement ayant 1 à 4 atomes de carbone et en particulier, méthyle et éthyle.

Les polymères fixants anioniques à groupements carboxyliques préférés selon l'invention sont :

A) les homo- ou copolymères d'acide acrylique ou méthacrylique ou leurs sels et en particulier les produits commercialisés sous les dénominations VERSICOL E ou K par la société ALLIED COLLOID et ULTRAHOLD par la société BASF. Les copolymères d'acide acrylique et d'acrylamide commercialisés sous la forme de leur sel de sodium sous les dénominations RETEN 421, 423 ou 425 par la Société HERCULES, les sels de sodium des acides polyhydroxycarboxyliques.

B) les copolymères des acides acrylique ou méthacrylique avec un monomère monoéthylénique tel que l'éthylène, le styrène, les esters vinyliques, les esters d'acide acrylique ou méthacrylique. Ces copolymères peuvent être greffés sur un polyalkylène glycol tel que le polyéthylène glycol et éventuellement réticulés. De tels polymères sont décrits en particulier dans le brevet français 1.222.944 et la demande allemande 2.330.956. On peut notamment citer les copolymères comportant dans leur chaîne un motif acrylamide éventuellement N-alkylé et/ou hydroxyalkylé tels que décrits notamment dans les demandes de brevets luxembourgeois 75370 et 75371 ou proposés sous la dénomination QUADRAMER par la Société AMERICAN CYANAMID. On peut également citer les copolymères d'acide acrylique et de méthacrylate d'alkyle en $C_1$-$C_4$ et les terpolymères de vinylpyrrolidone, d'acide (méth)acrylique et de (méth)acrylate d'alkyle en $C_1$-$C_{20}$ par exemple de lauryle (tel que celui commercialisé par la société ISP sous la dénomination ACRYLIDONE LM), de tertiobutyle (LUVIFLEX VBM 70 commercialisé par BASF) ou de méthyle (STEPANHOLD EXTRA commercialisé par STEPAN) et les terpolymères acide méthacrylique/ acrylate d'éthyle/ acrylate de tertiobutyle tel que le produit commercialisé sous la dénomination LUVIMER 100 P par la société BASF.

C) les copolymères dérivés d'acide crotonique tels que ceux comportant dans leur chaîne des motifs acétate ou propionate de vinyle et éventuellement d'autres monomères tels que esters allylique ou méthallylique, éther vinylique ou ester vinylique d'un acide carboxylique saturé linéaire ou ramifié à longue chaîne hydrocarbonée tels que ceux comportant au moins 5 atomes de carbone, ces polymères pouvant éventuellement être greffés et réticulés ou encore un ester vinylique, allylique ou méthallylique d'un acide carboxylique $\alpha$- ou $\beta$-cyclique. De tels polymères sont décrits entre autres dans les brevets français 1.222.944, 1.580.545, 2.265.782, 2.265.781, 1.564.110 et 2.439.798. Des produits commerciaux entrant dans cette classe sont les résines 28-29-30, 26-13-14 et 28-13-10 commercialisées par la société NATIONAL STARCH.

D) les copolymères dérivés d'acides ou d'anhydrides carboxyliques monoinsaturés en C4-C8 choisis parmi :

- les copolymères comprenant (i) un ou plusieurs acides ou anhydrides maléique, fumarique, itaconique et (ii) au moins un monomère choisis parmi les esters vinyliques, les éthers vinyliques, les halogénures vinyliques, les dérivés phénylvinyliques, l'acide acrylique et ses esters, les fonctions anhydrides de ces copolymères étant éventuellement monoestérifiées ou monoamidifiées ; De tels polymères sont décrits en particulier dans les brevets US 2.047.398, 2.723.248, 2.102.113, le brevet GB 839.805 et notamment ceux commercialisés sous les dénominations GANTREZ AN ou ES, AVANTAGE CP par la société ISP;
- les copolymères comprenant (i) un ou plusieurs anhydrides maléique, citraconique, itaconique et (ii) un ou plusieurs monomères choisis parmi les esters allyliques ou méthallyliques comportant éventuellement un ou plusieurs groupement acrylamide, méthacrylamide, $\alpha$-oléfine, esters acryliques ou méthacryliques, acides acrylique ou méthacrylique ou vinylpyrrolidone dans leur chaîne,

les fonctions anhydrides de ces copolymères étant éventuellement monoestérifiées ou monoamidifiées.

Ces polymères sont par exemple décrits dans les brevets français 2.350.384 et 2.357.241 de la demanderesse.

E) les polyacrylamides comportant des groupements carboxylates.

Les polymères comprenant les groupements sulfoniques sont des polymères comportant des motifs vinylsulfonique, styrène sulfonique, naphtalène sulfonique ou acrylamido alkylsulfonique.

Ces polymères peuvent être notamment choisis parmi :

- les sels de l'acide polyvinylsulfonique ayant un poids moléculaire moyen en poids compris entre environ 1.000 et 100.000 ainsi que les copolymères avec un comonomère insaturé tel que les acides acrylique ou méthacrylique et leurs esters ainsi que l'acrylamide ou ses dérivés, les éthers vinyliques et la vinylpyrrolidone;
- les sels de l'acide polystyrène sulfonique les sels de sodium ayant un poids moléculaire moyen en poids d'environ 500.000 et d'environ 100.000 commercialisés respectivement sous les dénominations Flexan 500 et Flexan 130 par National Starch. Ces composés sont décrits dans le brevet FR 2.198.719;
- les sels d'acides polyacrylamide sulfoniques ceux mentionnés dans le brevet US 4.128.631 et plus particulièrement l'acide polyacrylamidoéthylpropane sulfonique commercialisé sous la dénomination COSMEDIA POLYMER HSP 1180 par Henkel.

Selon l'invention, les polymères fixants anioniques sont de préférence choisis parmi les copolymères d'acide acrylique tels que les terpolymères acide acrylique / acrylate d'éthyle / N-tertiobutylacrylamide commercialisés notamment sous la dénomination ULTRAHOLD STRONG par la société BASF, les copolymères dérivés d'acide crotonique tels que les terpolymères acétate de vinyle / tertio-butyl benzoate de vinyle / acide crotonique et les terpolymères acide crotonique / acétate de vinyle/ néododécanoate de vinyle commercialisés notamment sous la dénomination Résine 28-29-30 par la société NATIONAL STARCH, les polymères dérivés d'acides ou d'anhydrides maléique, fumarique, itaconique avec des esters vinyliques, des éthers vinyliques, des halogénures vinyliques, des dérivés phénylvinyliques, l'acide acrylique et ses esters tels que les copolymères méthylvinyléther/anhydride maléique mono estérifiés commercialisés par exemple sous la dénomination GANTREZ par la société ISP, les copolymères d'acide méthacrylique et de méthacrylate de méthyle commercialisés sous la dénomination EUDRAGIT L par la société ROHM PHARMA, les copolymères d'acide méthacrylique/ méthacrylate de méthyle / acrylate d'alkyle en C1-C4 / acide acrylique ou méthacrylate d'hydroxyalkyle en C1-C4 commercialisés sous forme de dispersions sous la dénomination AMERHOLD DR 25 par la société AMERCHOL ou sous la dénomination ACUDYNE 255 par la société ROHM & HAAS, les copolymères d'acide méthacrylique et d'acrylate d'éthyle commercialisés sous la dénomination LUVIMER MAEX ou MAE par la société BASF et les copolymères acétate de vinyle/acide crotonique et les copolymères acétate de vinyle/acide crotonique greffés par du polyéthylèneglycol sous la dénomination ARISTOFLEX A par la société BASF.

Les polymères fixants anioniques les plus particulièrement préférés sont choisis parmi les copolymères méthylvinyléther / anhydride maléique mono estérifiés commercialisés sous la dénomination GANTREZ ES 425 par la société ISP, les terpolymères acide acrylique / acrylate d'éthyle / N-tertiobutylacrylamide commercialisés sous la dénomination ULTRAHOLD STRONG par la société BASF, les copolymères d'acide méthacrylique et de méthacrylate de méthyle commercialisés sous la dénomination EUDRAGIT L par la société ROHM PHARMA, les terpolymères acétate de vinyle / tertio-butyl benzoate de vinyle / acide crotonique et les terpolymères acide crotonique / acétate de vinyle / néododécanoate de vinyle commercialisés sous la dénomination Résine 28-29-30 par la société NATIONAL STARCH, les copolymères d'acide méthacrylique et d'acrylate d'éthyle commercialisés sous la dénomination LUVIMER MAEX OU MAE par la société BASF, les terpolymères vinylpyrrolidone / acide acrylique / méthacrylate de lauryle commercialisés sous la dénomination ACRYLIDONE LM par la société ISP.

Les polymères fixants amphotères utilisables conformément à l'invention peuvent être choisis parmi les polymères comportant des motifs B et C répartis statistiquement dans la chaîne polymère où B désigne un motif dérivant d'un monomère comportant au moins un atome d'azote basique et C désigne un motif dérivant d'un monomère acide comportant un ou plusieurs groupements carboxyliques ou sulfoniques ou bien B et C peuvent désigner des groupements dérivant de monomères zwittérioniques de carboxybétaïnes ou de sulfobétaïnes; B et C peuvent également désigner une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un radical hydrocarboné ou bien B et C font partie d'une chaîne d'un polymère à motif éthylène a,b-dicarboxylique dont l'un des groupements carboxyliques a été amené à réagir avec une polyamine comportant un ou plusieurs groupements amine primaire ou secondaire.

Les polymères fixants amphotères répondant à la définition donnée ci-dessus plus particulièrement préférés sont choisis parmi les polymères suivants :

1) les polymères résultant de la copolymérisation d'un monomère dérivé d'un composé vinylique portant un groupement carboxylique tel que plus particulièrement l'acide acrylique, l'acide méthacrylique, l'acide maléïque, l'acide alpha-chloracrylique, et d'un monomère basique dérivé d'un composé vinylique substitué contenant au moins un atome basique tel que plus particulièrement les dialkylaminoalkylméthacrylate et acrylate, les dialkylaminoalkylméthacrylamide et acrylamide. De tels composés sont décrits dans le brevet américain n° 3 836 537.

(2) les polymères comportant des motifs dérivant :

a) d'au moins un monomère choisi parmi les acrylamides ou les méthacrylamides substitués sur l'azote par un radical alkyle,

b) d'au moins un comonomère acide contenant un ou plusieurs groupements carboxyliques réactifs, et

c) au moins un comonomère basique tel que des esters à substituants amine primaire, secondaire, tertiaire et quaternaire des acides acrylique et méthacrylique et le produit de quaternisation du méthacrylate de diméthylaminoéthyle avec le sulfate de diméthyle ou diéthyle.

Les acrylamides ou méthacrylamides N-substitués plus particulièrement préférés selon l'invention sont les groupements dont les radicaux alkyle contiennent de 2 à 12 atomes de carbone et plus particulièrement le N-éthylacrylamide, le N-tertiobutyl acrylamide, le N-tertiooctyl acrylamide, le N-octylacrylamide, le N-décylacrylamide, le N-dodécylacrylamide ainsi que les méthacrylamides correspondants.

Les comonomères acides sont choisis plus particulièrement parmi les acides acrylique, méthacrylique, crotonique, itaconique, maléïque, fumarique ainsi que les monoesters d'alkyle ayant 1 à 4 atome de carbone des acides ou des anhydrides maléïque ou fumarique.

Les comonomères basiques préférés sont des méthacrylates d'amino-éthyle, de butyl aminoéthyle, de N,N'-diméthylaminoéthyle, de N-tertio-butyl-aminoéthyle.

On utilise particulièrement les copolymères dont la dénomination CTFA (4ème Ed., 1991) est Octylacrylamide/acrylates/butylaminoethylmethacrylate copolymer tels que les produits commercialisés sous la dénomination AMPHOMER ou LOVOCRYL 47 par la société NATIONAL STARCH.

(3) les polyamino amides réticulés et alcoylés partiellement ou totalement dérivant de poyaminoamides de formule générale :

$$-\left[ CO - R_{10} - CO - Z \right]- \qquad \text{(III)}$$

dans laquelle $R_{10}$ représente un radical divalent dérivé d'un acide dicarboxylique saturé, d'un acide aliphatique mono ou dicarboxylique à double liaison éthylénique, d'un ester d'un alcanol inférieur ayant 1 à 6 atome de carbone de ces acides ou d'un radical dérivant de l'addition de l'un quelconque desdits acides avec une amine bis primaire ou bis secondaire, et Z désigne un radical d'une polyalkylène-polyamine bis-primaire, mono ou bis-secondaire et de préférence représente :

a) dans les proportions de 60 à 100 moles %, le radical

$$-NH-\left[ (CH_2)_x - NH \right]_p- \qquad \text{(IV)}$$

où x=2 et p=2 ou 3, ou bien x=3 et p=2

ce radical dérivant de la diéthylène triamine, de la triéthylène tétraamine ou de la dipropylène triamine;

b) dans les proportions de 0 à 40 moles % le radical (IV) ci-dessus, dans lequel x=2 et p=1 et qui dérive de l'éthylènediamine, ou le radical dérivant de la pipérazine :

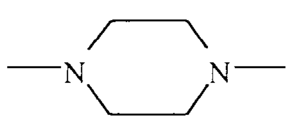

c) dans les proportions de 0 à 20 moles % le radical $-NH-(CH_2)_6-NH-$dérivant de l'hexaméthylènediamine, ces polyaminoamines étant réticulées par addition d'un agent réticulant bifonctionnel choisi parmi les épihalohydrines, les diépoxydes, les dianhydrides, les dérivés bis insaturés, au moyen de 0,025 à 0,35 mole d'agent réticulant par groupement amine du polyaminoamide et alcoylés par action d'acide acrylique, d'acide chloracétique ou d'une alcane sultone ou de leurs sels.

Les acides carboxyliques saturés sont choisis de préférence parmi les acides ayant 6 à 10 atomes de carbone tels que l'acide adipique, triméthyl-2,2,4-adipique et triméthyl-2,4,4-adipique, téréphtalique, les acides à double liaison éthylénique comme par exemple les acides acrylique, méthacrylique, itaconique.

Les alcanes sultones utilisées dans l'alcoylation sont de préférence la propane ou la butane sultone, les sels des agents d'alcoylation sont de préférence les sels de sodium ou de potassium.

(4) les polymères comportant des motifs zwittérioniques de formule :

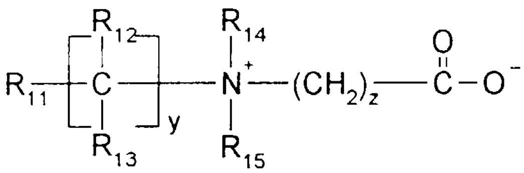

(V)

dans laquelle $R_{11}$ désigne un groupement insaturé polymérisable tel qu'un groupement acrylate, méthacrylate, acrylamide ou méthacrylamide, y et z représente un nombre entier de 1 à 3, $R_{12}$ et $R_{13}$ représentent un atome d'hydrogène, méthyle, éthyle ou propyle, $R_{14}$ et $R_{15}$ représentent un atome d'hydrogène ou un radical alkyle de telle façon que la somme des atomes de carbone dans $R_{14}$ et $R_{15}$ ne dépasse pas 10.

Les polymères comprenant de telles unités peuvent également comporter des motifs dérivés de monomères non zwittérioniques tels que l'acrylate ou le méthacrylate de diméthyl ou diéthylaminoéthyle ou des alkyle acrylates ou méthacrylates, des acrylamides ou méthacrylamides ou l'acétate de vinyle.

A titre d'exemple, on peut citer le copolymère de méthacrylate de méthyle / diméthyl carboxyméthylammonio éthylméthacrylate de méthyle tel que le produit commercialisé sous la dénomination DIAFORMER Z301 par la société SANDOZ.

(5) les polymères dérivés du chitosane comportant des motifs monomères répondant aux formules suivantes :

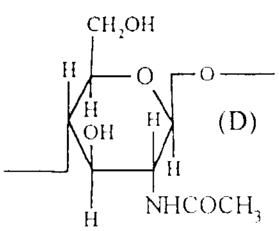

(D)

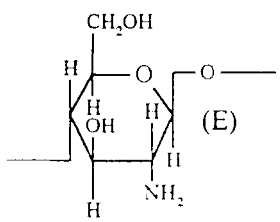

(E)

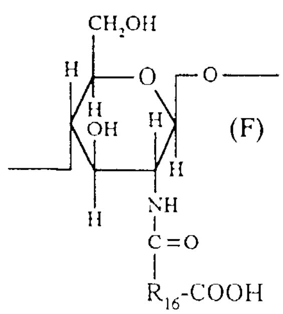

(F)

le motif D étant présent dans des proportions comprises entre 0 et 30%, le motif E dans des proportions comprises entre 5 et 50% et le motif F dans des proportions comprises entre 30 et 90%, étant entendu que dans ce motif F, $R_{16}$ représente un radical de formule :

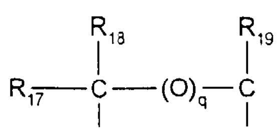

dans laquelle si q=0, $R_{17}$, $R_{18}$ et $R_{19}$, identiques ou différents, représentent chacun un atome d'hydrogène, un reste méthyle, hydroxyle, acétoxy ou amino, un reste monoalcoylamine ou un reste dialcoylamine éventuellement interrompus par un ou plusieurs atomes d'azote et/ou éventuellement substitués par un ou plusieurs groupes amine, hydroxyle, carboxyle, acoylthio, sulfonique, un reste alcoylthio dont le groupe alcoyle porte un reste amino, l'un au moins des radicaux $R_{17}$, $R_{18}$ et $R_{19}$ étant dans ce cas un atome d'hydrogène ;
ou si q=1, $R_{17}$, $R_{18}$ et $R_{19}$ représentent chacun un atome d'hydrogène, ainsi que les sels formés par ces composés avec des bases ou des acides.

(6) Les polymères dérivés de la N-carboxyalkylation du chitosane comme le N-carboxyméthyl chitosane ou le N-carboxybutyl chitosane commercialisé sous la dénomination "EVALSAN" par la société JAN DEKKER.

(7) Les polymères répondant à la formule générale (VI) sont par exemple décrits dans le brevet français 1 400 366 :

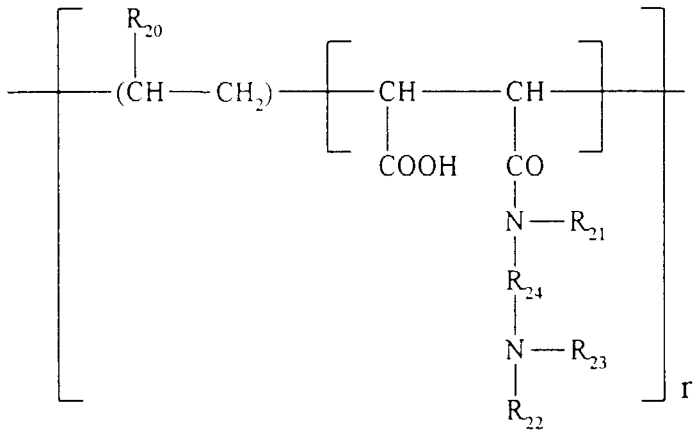

(VI)

dans laquelle $R_{20}$ représente un atome d'hydrogène, un radical $CH_3O$, $CH_3CH_2O$, phényle, $R_{21}$ désigne l'hydrogène ou un radical alkyle inférieur tel que méthyle, éthyle, $R_{22}$ désigne l'hydrogène ou un radical alkyle inférieur tel que méthyle, éthyle, $R_{23}$ désigne un radical alkyle inférieur tel que méthyle, éthyle ou un radical répondant à la formule : $-R_{24}-N(R_{22})_2$, $R_{24}$ représentant un groupement $-CH_2-CH_2-$ , $-CH_2-CH_2-CH_2-$, $-CH_2-CH(CH_3)-$ , $R_{22}$ ayant les significations mentionnées ci-dessus,

ainsi que les homologues supérieurs de ces radicaux et contenant jusqu'à 6 atomes de carbone.

(8) Des polymères amphotères du type -D-X-D-X choisis parmi:

a) les polymères obtenus par action de l'acide chloracétique ou le chloracétate de sodium sur les composés comportant au moins un motif de formule :

-D-X-D-X-D- (VII)

où D désigne un radical

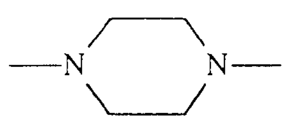

et X désigne le symbole E ou E', E ou E' identiques ou différents désignent un radical bivalent qui est un radical alkylène à chaîne droite ou ramifiée comportant jusqu'à 7 atomes de carbone dans la chaîne principale non substituée ou substituée par des groupements hydroxyle et pouvant comporter en outre des atomes d'oxygène, d'azote, de soufre, 1 à 3 cycles aromatiques et/ou hétérocycliques; les atomes d'oxygène, d'azote et de soufre étant présents sous forme de groupements éther, thioéther, sulfoxyde, sulfone, sulfonium, alkylamine, alkénylamine, des groupements hydroxyle, benzylamine, oxyde d'amine, ammonium quaternaire, amide, imide, alcool, ester et/ou uréthanne.

b) Les polymères de formule :

-D-X-D-X- (VII')

où D désigne un radical

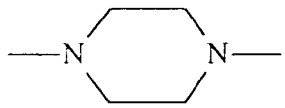

et X désigne le symbole E ou E' et au moins une fois E'; E ayant la signification indiquée ci-dessus et E' est un radical bivalent qui est un radical alkylène à chaîne droite ou ramifiée ayant jusqu'à 7 atomes de carbone dans la chaîne principale, substitué ou non par un ou plusieurs radicaux hydroxyle et comportant un ou plusieurs atomes d'azote, l'atome d'azote étant substitué par une chaîne alkyle interrompue éventuellement par un atome d'oxygène et comportant obligatoirement une ou plusieurs fonctions carboxyle ou une ou plusieurs fonctions hydroxyle et bétaïnisées par réaction avec l'acide chloracétique ou du chloracétate de soude.

(9) les copolymères alkyl(C1-C5)vinyléther / anhydride maléique modifié partiellement par semiamidification avec une N,N-dialkylaminoalkylamine telle que la N,N-diméthylaminopropylamine ou par semiestérification avec une N,N-dialcanolamine. Ces copolymères peuvent également comporter d'autres comonomères vinyliques tels que le vinylcaprolactame.

Les polymères fixants amphotères particulièrement préférés selon l'invention sont ceux de la famille (3) tels que les copolymères dont la dénomination CTFA est Octylacrylamide/acrylates/butylaminoethylmethacrylate copolymer tels que les produits commercialisés sous les dénominations AMPHOMER, AMHOMER LV 71 ou LOVOCRYL 47 par la société NATIONAL STARCH et ceux de la famille (4) tels que les copolymère de méthacrylate de méthyle / diméthyl carboxyméthylammonio éthylméthacrylate de méthyle par exemple commercialisé sous la dénomination DIAFORMER Z301 par la société SANDOZ.

Les polymères fixants anioniques ou amphotères peuvent être si nécessaire neutralisés partiellement ou totalement. Les agents de neutralisation sont par exemple la soude, la potasse, l'amino-2 méthyl-2 propanol-1, la monoéthanolamine, la triéthanolamine ou la triisopropanolamine, les acides minéraux ou organiques tels que l'acide chlorhydrique ou l'acide citrique.

De manière avantageuse, le solvant approprié contient au moins 50 % en volume d'alcool, de préférence au moins 70 % en volume d'alcool. Par alcool on entend selon l'invention un alcool aliphatique en $C_1$-$C_4$, de préférence l'éthanol.

Le propulseur est constitué par les gaz comprimés ou liquéfiés usuellement employés pour la préparation de compositions aérosols. On emploiera de manière préférentielle l'air, le gaz carbonique ou l'azote comprimés, ou encore un gaz soluble ou non dans la composition tel que le diméthyl éther, les hydrocarbures et leurs mélanges.

En fonction de la composition aérosol (jus + propulseur), l'homme du métier saura sélectionner le moyen de distribution approprié pour obtenir les caractéristiques de débit en matière sèche et de pouvoir mouillant recherchées.

Les caractéristiques particulières définies ci-dessus ($C_{MS}$ et phase), peuvent être obtenues en sélectionnant les moyens de distribution appropriés et/ou en agissant sur la formulation.

Les valves appropriées pour les compositions particulières ci-dessus sont notamment des valves droites avec un gicleur de diamètre compris entre 0,35 et 0,60 mm, de préférence compris entre 0,40 et 0,50 mm, avantageusement sans restriction interne ni prise de gaz additionnelle. Il s'agit en particulier des valves commercialisées sous la dénomibnation COSTER T104 RA36/0/4 par la société COSTER, ou de la valve Précision Expérimentale 15130 constituée par un gicleur et un corps de valve de 0,46 mm de diamètres sans prise de gaz additionnelle de la société Précision.

Les diffuseurs appropriés pour les compositions particulières ci-dessus sont en particulier les boutons poussoirs commercialisés sous la dénomination Précision 216903-40AD29 par la société Précision.

La présente invention concerne également un procédé de traitement des fibres kératiniques, dans lequel on applique sur lesdites fibres une composition comprenant un matériau fixant ayant une température de transition vitreuse (Tg) supérieure ou égale à 30 °C, au moyen d'un dispositif approprié pour obtenir un débit en matière sèche compris entre environ 4 et 17 mg/s et un pouvoir mouillant supérieur ou égal à 50 mg/s.

Les exemples ci-après permettent d'illustrer l'invention sans toutefois en limiter la portée.

**<u>Exemple 1</u> : Importance du « pouvoir mouillant » pour laquer**

On prépare les deux dispositifs aérosol suivants:

Dispositif 1 (selon l'invention)

On prépare le jus suivant:

| Composition A | |
|---|---|
| Terpolymère acide acrylique / acrylate d'éthyle / N-tertiobutylacrylamide commercialisé sous la dénomination ULTRAHOLD STRONG par la société BASF | 3,10 g |
| 2-Amino-2-méthyl-propan-1-ol qs neutralisation | |
| Ethanol qs | 100,00 g |

On introduit 65g de ce jus dans un flacon aérosol que l'on équipe d'une valve Précision Expérimentale 15130, puis on ajoute 35 g de diméthyl éther comme propulseur, et un bouton poussoir Précision 216903-40AD29.

Les caractéristiques de ce dispositif sont les suivantes:

| | |
|---|---|
| Tg du matériau fixant | 51 °C |
| $D_{MS}$ | 11 mg/s |
| Pouvoir mouillant | 55 mg/s |
| $D_{CA}$ | 550 mg/s |

Dispositif 2 (comparatif)

On prépare le jus suivant:

| Composition B | |
|---|---|
| Terpolymère acide acrylique / acrylate d'éthyle / N-tertiobutylacrylamide commercialisé sous la dénomination ULTRAHOLD STRONG par la société BASF | 4,28 g |
| 2-Amino-2-méthyl-propan-1-ol qs neutralisation | |
| Ethanol qs | 100,00 g |

On introduit 35g de ce jus et 20 g de pentane dans un flacon aérosol que l'on équipe d'une valve Précision P155/590 (commercialisée par la société Précision), puis on ajoute 43 g de diméthyl éther comme propulseur, et un bouton poussoir Précision 21 6943-40 (commercialisé par la société Précision).

Les caractéristiques de ce dispositif sont les suivantes:

| | |
|---|---|
| Tg du matériau fixant | 51 °C |
| $D_{MS}$ | 11 mg/s |
| Pouvoir mouillant | 10 mg/s |
| $D_{CA}$ | 700 mg/s |

On teste ces deux dispositifs sur têtes (20 s par tête). Les tests montrent que seul le dispositif selon l'invention permet d'obtenir un effet de fixation cosmétique recherché. Par contre, le dispositif 2 (comparatif) ne permet pas d'obtenir un résultat de fixation cosmétique satisfaisant.

**Exemple 2 : Importance de la « Tg » pour obtenir de bonnes propriétés cosmétiques**

On prépare les jus suivants:

| Composition C | |
|---|---|
| VA / vinyl butyl benzoate / crotonates copolymer (CTFA) | 4,28 g |
| 2-Amino-2-méthyl-propan-1-ol qs neutralisation | |
| Monométhyléther de tripropylène glycol | 0,29 g |
| Ethanol qs | 100,00 g |

| Composition D | |
|---|---|
| VA / vinyl butyl benzoate / crotonates copolymer (CTFA)<br>2-Amino-2-méthyl-propan-1-ol qs neutralisation<br>Ethanol qs | 4,28 g<br><br><br>100,00 g |

On prépare les dispositifs aérosols 3 et 4 en introduisant dans des flacons aérosol 65 g des compositions C et D, respectivement. On équipe les deux flacons d'une valve Précision Expérimentale 15130, puis on ajoute 35 g de diméthyl éther comme propulseur, et un bouton poussoir Précision 216903-40AD29.

Pour les deux dispositifs, le $D_{MS}$ est de 14,3 mg/s et le pouvoir mouillant de 62 mg/s.

Les Tg des matériaux fixants dans les deux dispositifs sont les suivants:

| | |
|---|---|
| Tg dispositif 3 (comparatif) | 23 °C |
| Tg dispositif 4 (selon l'invention) | 41 °C |

On compare les performances des deux dispositifs en pulvérisant sur 10 têtes les deux compositions par demi-têtes. Dans tous les cas, les pouvoirs fixants sont satisfaisants et comparables. En revanche, le toucher est plus agréable du côté traité par le dispositif 4 selon l'invention par rapport au côté traité par le dispositif 3 comparatif, de même que le démêlage est plus aisé de ce même côté, ainsi que le toucher après démêlage est jugé plus agréable.

**Exemple 3: Importance du « $D_{MS}$» pour obtenir de bonnes propriétés cosmétiques**

On prépare les deux jus suivants:

| Composition E | |
|---|---|
| VA / vinyl butyl benzoate / crotonates copolymer (CTFA)<br>2-Amino-2-méthyl-propan-1-ol qs neutralisation<br>Ethanol qs | 4,00 g<br><br><br>100,00 g |

| Composition F | |
|---|---|
| VA / vinyl butyl benzoate / crotonates copolymer (CTFA)<br>2-Amino-2-méthyl-propan-1-ol qs neutralisation<br>Ethanol qs | 5,00 g<br><br><br>100,00 g |

On prépare les dispositifs aérosols 5 et 6 en introduisant dans des flacons aérosol 65 g des compositions E et F, respectivement. On équipe les deux flacons d'une valve Précision Expérimentale 15130, puis on ajoute 35 g de diméthyl éther comme propulseur, et un bouton poussoir Précision 216903-40AD29.

Pour les deux dispositifs, le Tg du matériau fixant est de 41 °C et le pouvoir mouillant de 62 mg/s.

Les $D_{MS}$ des matériaux fixants dans les deux dispositifs sont les suivants:

| | |
|---|---|
| $D_{MS}$ dispositif 5 (selon l'invention) | 15,0 mg/s |
| $D_{MS}$ dispositif 6 (comparatif) | 18,5 mg/s |

On compare les performances des deux dispositifs en pulvérisant sur 10 têtes les deux compositions par demi-têtes. Dans tous les cas, les pouvoirs fixants sont satisfaisants et comparables. En revanche, le toucher est plus agréable du côté traité par le dispositif 5 selon l'invention par rapport au côté traité par le dispositif 6 comparatif, de même que le démêlage est plus aisé de ce même côté, ainsi que le toucher après démêlage est jugé plus agréable.

**Exemple 4 : Comparaisons**

Les propriétés de démêlage obtenues avec le dispositif 4 (65 g de la composition D, valve Précision Expérimentale 15130, 35 g de diméthyl éther, bouton poussoir Précision 216903-40AD29) ont été comparées avec celles de dispositifs commerciaux.

| Dispositif | Tg | $D_{MS}$ | P. Mouillant | Démêlage |
|---|---|---|---|---|
| Dispositif 4 | 41 °C | 14,3 mg/s | 62 mg/s | +++ |
| *Pantène Pro V® | 50 °C | 17,4 mg/s | > 190 mg/s | ++ |
| **Aqua Net® | 23 °C | 16,8 mg/s | 190 mg/s | + |
| **Elnett® Fixation Forte | < 30 °C | > 20 mg/s | 40 mg/s | ++ |

* commercialisé par PROCTER & GAMBLE

** commercialisé par FABERGE

*** commercialisé par L'OREAL

\+ à +++ représentent une échelle croissante des qualités de démêlage des différentes compositions.

Les résultats du tableau ci-dessus sont conformes à ceux observés dans les exemples 1 à 3, où seul le dispositif répondant aux trois caractéristiques de « Tg », « $D_{MS}$ » et « pouvoir mouillant » selon l'invention permet d'obtenir à la fois une fixation forte et des propriétés cosmétiques améliorées.

**Revendications**

**1.** Dispositif aérosol constitué par un récipient contenant une composition aérosol constituée d'une part par une phase liquide (ou jus) contenant au moins un matériau fixant dans un solvant approprié et d'autre part un propulseur, et un moyen de distribution de ladite composition aérosol, caractérisé en ce que le matériau fixant a une température de transition vitreuse (Tg) supérieure ou égale à 30 °C, et en ce que le dispositif est approprié pour obtenir un débit en matière sèche compris entre environ 4 et 17 mg/s et un pouvoir mouillant supérieur ou égal à 50 mg/s.

**2.** Dispositif selon la revendication 1, caractérisé en ce qu'il est approprié pour obtenir un débit en matière sèche inférieur à 16 mg/s, de préférence compris entre 6 et 15 mg/s.

**3.** Dispositif selon l'une des revendications 1 ou 2, caractérisé en ce qu'il est approprié pour obtenir un pouvoir mouillant compris entre 50 mg/s et 125 mg/s.

**4.** Dispositif selon l'une des revendications 1 à 3, caractérisé en ce que la concentration en matière sèche ($C_{MS}$) est comprise entre 0,4 et 5% en poids par rapport au poids total de la composition aérosol (jus + propulseur), de préférence comprise entre 0,6 et 3,25 % en poids.

**5.** Dispositif selon l'une des revendications 1 à 4, caractérisé en ce que le débit en composition aérosol ($D_{CA}$) est compris entre 500 et 700 mg/s, de préférence voisin de 550 mg/s.

**6.** Dispositif selon l'une des revendications 1 à 5, caractérisé en ce que le rapport pondéral jus/propulseur est supérieur à 1, de préférence compris entre 1,2 et 3.

**7.** Dispositif selon l'une des revendications 1 à 6, caractérisé en ce que le matériau fixant est essentiellement constitué par au moins un polymère fixant.

**8.** Dispositif selon la revendication 7, caractérisé en ce que le polymère fixant est choisi parmi les polymères anioniques, cationiques, amphotères, non ioniques et leurs mélanges.

**9.** Dispositif selon l'une des revendications 1 à 8, caractérisé en ce que le solvant approprié contient au moins 50 % en volume d'alcool, de préférence au moins 70 % en volume d'alcool.

**10.** Procédé de traitement des fibres kératiniques, caractérisé en ce qu'on applique sur lesdites fibres une composition comprenant un matériau fixant ayant une température de transition vitreuse (Tg) supérieure ou égale à 30 °C, au

moyen d'un dispositif approprié pour obtenir un débit en matière sèche compris entre environ 4 et 17 mg/s et un pouvoir mouillant supérieur ou égal à 50 mg/s.

Office européen des brevets

# RAPPORT DE RECHERCHE EUROPEENNE

Numéro de la demande
EP 97 40 2847

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.6) |
|---|---|---|---|
| X | WO 95 00105 A (EASTMAN CHEMICAL COMPANY) 5 janvier 1995<br>* revendications 1-3,11,12 *<br>* page 6, ligne 25-28 *<br>* exemples 1-10 *<br>--- | 1,4,6-10 | A61K7/00<br>A61K7/06 |
| X | WO 95 33437 A (EASTMAN CHEMICAL COMPANY) 14 décembre 1995<br>* revendications 1-6,15-17 *<br>* page 5, ligne 27-32 *<br>* exemple 2 *<br>--- | 1,4,6-10 | |
| X | WO 95 03776 A (MINNESOTA MINING AND MANUFACTURING) 9 février 1995<br>* revendications 1,11,12 *<br>* page 45, ligne 25-36 *<br>* page 46, ligne 15-27 *<br>--- | 1,7-10 | |
| X | WO 96 32918 A (PROCTER & GAMBLE) 24 octobre 1996<br>* revendications 1,10 *<br>* page 5, ligne 18-35 *<br>* page 13, ligne 5-13 *<br>* page 16, ligne 35 - page 17, ligne 16 *<br>--- | 1,4,6-10 | DOMAINES TECHNIQUES RECHERCHES (Int.Cl.6)<br>A61K |
| X | US 5 266 303 A (G.L.MYERS E.A.) 30 novembre 1993<br>* revendication 1 *<br>* colonne 5, ligne 8-18 *<br>* exemple 2 * | 1,4,6-10 | |
| D,X | & WO 94 12148 A (EASTMAN KODAK)<br>----- | 1,4,6-10 | |

Le présent rapport a été établi pour toutes les revendications

| Lieu de la recherche | Date d'achèvement de la recherche | Examinateur |
|---|---|---|
| LA HAYE | 12 mars 1998 | Peeters, J |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)